# EUROPEAN PATENT APPLICATION

(11) **EP 4 438 106 A1**
(43) Date of publication of application: **02.10.2024**
(21) Application number: 22921552.0
(22) Date of filing: 21.10.2022
(51) Int. Cl.: A61N 1/36, A61N 1/05, A61B 5/388, A61B 5/262, A61B 5/294, A61B 5/263

(54) **MICRONEEDLE AND NEURAL INTERFACE SYSTEM**

(30) Priority: 21.01.2022 CN 202210072843
(71) Applicant: Wuhan Neuracom Technology Development Co., Ltd, Wuhan, Hubei 430200 (CN)
(72) Inventor: HUANG, Li, Wuhan, Hubei 430200 (CN); HUANG, Cheng, Wuhan, Hubei 430200 (CN); JI, Junwang, Wuhan, Hubei 430200 (CN); GAO, Jianfei, Wuhan, Hubei 430200 (CN); MA, Zhanfeng, Wuhan, Hubei 430200 (CN)
(74) Representative: Leihkauf, Steffen Falk
(86) International application number: PCT/CN2022/126565
(87) International publication number: WO 2023/138128

(57) **Abstract**

Disclosed are a microneedle and a neural interface system. The microneedle comprises at least one microneedle body, wherein the microneedle body comprises a lining plate (5) and at least one body electrode (1), and the lining plate (5) is of an arc-shaped structure. The microneedle body has the form of an arc-shaped structure and can wrap an optic nerve (6), so as to facilitate the reading of an optic nerve signal, or stimulate the optic nerve (6) by means of the body electrode (1) so as to generate artificial vision in a visual center.

## Description

### TECHNICAL FIELD

The present application relates to the field of biomedical instruments. More specifically, the present application relates to a microneedle and a neural interface system.

### BACKGROUND ART

Similar to artificial cochlea, visual repair is to convert, by an *in-vitro* camera, an image into an electrical signal in a certain encoding form, and directly stimulate vision-related nerves by an electrode system implanted in body to restore, improve and reconstruct the visual function of the blind. A visual pathway includes an optical path, a retina, an optic nerve, and a visual cortex. Currently, visual repair apparatuses are studied mainly from two directions of stimulating cortex and stimulating retina. In subretinal or extraretinal stimulation methods, a photodiode array is implanted in the retina between a bipolar cell layer and retinal pigment epithelium.

Currently, most optic nerve implant electrodes are patch-type electrodes, and an optic nerve implant electrode patch can only collect a signal of a nerve cluster, but cannot realize action potential of a single neuron cell.

### SUMMARY

The present application aims at providing a microneedle and a neural interface system, which can read an optic nerve signal or stimulate the optic nerve by a microelectrode, so as to generate artificial vision in the visual center.

In order to achieve these objectives and other advantages of the present application, a microneedle is provided, including: at least one microprobe, wherein the microprobe includes a lining plate of an arc-shaped structure and at least one microelectrode.

In one of the embodiments, the microneedle includes at least one integrated circuit chip, and the at least one microprobe and the at least one integrated circuit chip are in one-to-one correspondence.

In one of the embodiments, when the at least one microprobe is in the number of at least two, the integrated circuit chip is located between two adjacent microprobes.

In one of the embodiments, the at least one integrated circuit chip is each provided thereon with at least one welding spot, the lining plate is provided thereon with at least one welding spot, and the integrated circuit chip and the lining plate are bonded through the at least one welding spot.

In one of the embodiments, the at least one microelectrode is a Utah electrode, a Michigan electrode or an optogenetic electrode.

In one of the embodiments, the at least one microelectrode is located at an inner side of the lining plate.

In one of the embodiments, the at least one microelectrode is each provided thereon with at least one microelectrode point.

In order to achieve these objectives and other advantages of the present application, a neural interface system is provided, including the microneedle according to the present application and an attachment unit,
the microneedle is used for stimulating an optic nerve and/or collecting an optic nerve signal; and
the attachment unit is attached to a posterior wall of an eyeball.

In one of the embodiments, the microneedle and the attachment unit are connected by a connecting line.

In one of the embodiments, the attachment unit is made from a biocompatible material.

The present application at least includes the following beneficial effects: the microneedle can read the optic nerve signal or stimulate the optic nerve by the at least one microelectrode, so as to generate artificial vision in the visual center.

Further, each microneedle has a plurality of microelectrode points, and realizes synchronous detection and recording of the plurality of electrode points, improving spatial resolution and signal accuracy.

Other advantages, objectives and characteristics of the present application will be partly embodied in the following description, and partly understood by those skilled in the art by studying and practicing the present application.

### BRIEF DESCRIPTION OF DRAWINGS

FIG. 1 is a structural schematic diagram of a neural interface system provided in embodiments of the present application;
FIG. 2 is a schematic diagram of a microneedle being implanted into an optic nerve provided in embodiments of the present application;
FIG. 3 is a schematic diagram of a structure of another neural interface system (having two attachment units) provided in the present application; and
FIG. 4 is a schematic diagram of a structure of a further neural interface system (having two attachment units) provided in the present application.

Illustration of reference signs: 1 microelectrode, 2 connecting line, 3 attachment unit, 4 integrated circuit chip, 5 lining plate, 6 optic nerve.

### DETAILED DESCRIPTION OF EMBODIMENTS

The present application is further described in detail below in combination with drawings, so that those skilled in the art could implement the present application according to the written description.

In order to make the objectives, technical solutions and advantages of the present application clearer and more definite, the present application is further described in detail below with reference to the drawings and embodiments. It should be understood that the specific embodiments described herein are merely used for explaining the present application, but not intended to limit the present application.

It should be noted that if directional indications (such as upper, lower, left, right, front, back...) are involved in the embodiments in the present application, the directional indications are only used for explaining relative positional relations, movement conditions or the like between various components in some particular posture, and if the particular posture changes, the directional indications will also change accordingly.

### Embodiment 1:

The present embodiment provides a microneedle, including at least one microprobe, wherein the microprobe includes a lining plate of an arc-shaped structure and at least one microelectrode. The microneedle further includes at least one integrated circuit chip, wherein the at least one microprobe and the at least one integrated circuit chip are in one-to-one correspondence. In the above, the microelectrode is a Utah electrode, a Michigan electrode or an optogenetic electrode, and the microelectrode is provided thereon with at least one microelectrode point, for receiving stimulation or collecting a nerve signal. The microelectrode is located at an inner side of the lining plate. The inner side of the lining plate refers to a side facing an arc-shaped opening.

In an optional embodiment, when the microprobe is in the number of at least two, the integrated circuit chip is located between two adjacent microprobes.

Further, the integrated circuit chip is provided thereon with at least one welding spot, the lining plate is provided thereon with at least one welding spot, and the integrated circuit chip and the lining plate are bonded through the welding spot(s).

In the present embodiment, the microprobe is of an arc-shaped structure and can wrap an optic nerve, so as to facilitate the reading of an optic nerve signal, or stimulate the optic nerve by means of the microelectrode(s), so as to generate artificial vision in the visual center.

### Embodiment 2:

The present embodiment further provides a neural interface system, wherein the neural interface system includes the microneedle as described in Embodiment 1 and an attachment unit, and wherein the microneedle is used for stimulating an optic nerve and/or collecting an optic nerve signal; and the attachment unit is attached to a posterior wall of an eyeball.

In the present embodiment, the microneedle and the attachment unit are connected by a connecting line. In the above, the connecting line is an insulated line made from a biocompatible material; and the attachment unit is made from a biocompatible material, wherein the biocompatible materials include PDMS, polyimide, metal or alloy.

Structures of the microneedle and the neural interface system are described in detail below.

### Embodiment 3:

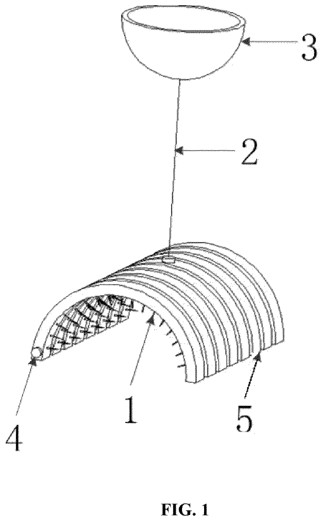

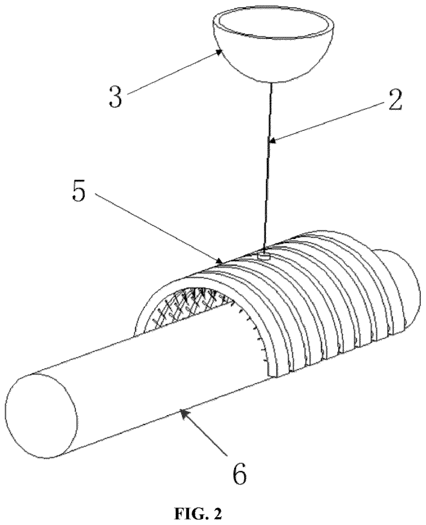

As shown in FIGS. 1 and 2, the present application provides a neural interface system, including: a microneedle and an attachment unit 3, wherein the microneedle is connected with the attachment unit 3 by a connecting line 2; the attachment unit 3 is attached to a posterior wall of an eyeball, and does not interfere with an optic nerve 6; and the microneedle is manufactured through a MEMS process. The microneedle includes at least one microprobe. The microprobe includes at least one microelectrode 1, wherein the microelectrode 1 is used for stimulating the optic nerve 6, and each microelectrode 1 has a plurality of microelectrode points.

In the above, the microelectrode 1 is used for detecting a nerve signal and sensing a weak-electricity stimulatory signal of the optic nerve 6, and is also used for sending a stimulatory signal to the optic nerve 6, wherein the stimulatory signal is a visual reproduction signal converted from an image signal. The attachment unit 3 is made from a flexible material, which may be silica gel or other flexible materials. The attachment unit 3 is in a semi-spherical shape. A diameter of the attachment unit may be adaptively adjusted according to an eyeball size of a patient. During operation, the attachment unit is attached to a posterior wall of the eyeball, and then the whole apparatus is fixed and adsorbed by means of stitching.

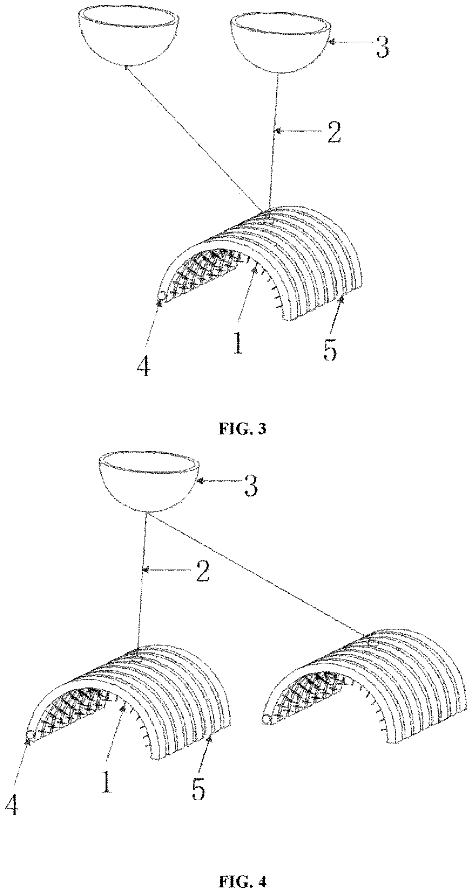

In an embodiment of the present application, when a patient suffering from binocular blindness is undergoing an operation using the neural interface system, as shown in FIG. 3, a structural form of the neural interface system is composed of two attachment units and one microneedle, wherein the two attachment units are both connected to one microneedle by the connecting lines, the two attachment units 3 are both attached to the posterior walls of the eyeballs, and do not interfere with the optic nerve 6, so that two blind eyeballs interact with the optic nerve corresponding to one microneedle. A structural form of the microneedle is the same as that in the preceding embodiment.

In another embodiment of the present application, in order to better restore the effective visual function of patients, a plurality of microneedles may be provided, to synchronously detect and sense nerve signals of multiple optic nerves and weak-electricity stimulatory signals. As shown in FIG. 4, the structural form of the neural interface system is composed of one attachment unit and two microneedles, wherein the microneedles are each connected with the one attachment unit by the connecting line, and two microprobes are provided at two different optic nerves, so as to realize interaction between one eyeball and multiple optic nerve signals.

The present application further provides another embodiment. In order to better restore the effective visual function of patients, multiple groups of microprobes may be provided to synchronously detect and sense nerve signals and weak-electricity stimulatory signals of one optic nerve in different positions. The structural form of the neural interface system is composed of one attachment unit and multiple groups of microprobes, wherein the multiple groups of microprobes are all connected with the one attachment unit by the connecting lines, and the multiple groups of microprobes are fixed at intervals in different positions of the same optic nerve, so as to realize interaction between one eyeball and signals of one optic nerve in different positions.

Some embodiments of the present application provide a method for mounting and connecting a microprobe and an integrated circuit chip, for example, the microprobe is bonded to the integrated circuit chip by a bonding process. Wires of several microelectrodes 1 are integrated into one wire, which is then connected with the integrated circuit chip, and the integrated circuit chip ensures the reading of a nerve signal and input of a weak-electricity stimulatory signal, thereby realizing transmission of signals. In the above, the integrated circuit chip may be a low-power-consumption integrated circuit chip 4.

In order to ensure the contact between the microelectrode 1 and the optic nerve 6, in some embodiments of the present application, several microelectrodes 1 are fixed on the lining plate 5 in an array, and probing ends of the several microelectrodes 1 protrude from one side of the lining plate 5. The microelectrodes 1 are ensured to protrude from the lining plate 5, so as to probe the optic nerve 6. The probing ends of the microelectrodes 1 are of a needle-like structure. In the above, the several microneedles may be provided to protrude from the lining plate by the same length, or may be provided to have gradually increased or gradually decreased lengths.

The present technical solution may further include the following technical details so as to better achieve the technical effects: the lining plate 5 in the microprobe is of an arc-shaped structure, and as the optic nerve 6 fiber is cylindrical, the providing the lining plate 5 to be arc-shaped better facilitates attached implantation of multi-contact microprobe.

In some embodiments of the present application, the connecting line 2 is a flexible connecting line, which facilitates flexible fixation of the microneedle and the attachment unit 3 between the optic nerve 6 and the eyeball.

In some embodiments of the present application, the microelectrode 1 collects a human nerve signal, and essentially, it is the microelectrode points on the microelectrode 1 that collect the human nerve signal. In order to collect the action potential of a single neuron cell, a plurality of microelectrode points are provided on the microelectrode 1, thus realizing simultaneous collection of a single neuron cell by a plurality of microelectrode points, that is, synchronous detection and recording of the plurality of electrode points. Meanwhile, the signal collection efficiency, spatial resolution and signal accuracy are also improved. In order to avoid interference between adjacent microelectrode points, the plurality of microelectrode points are staggered at intervals.

In the above, it should be noted that the plurality of microelectrode points are manufactured by the MEMS process, and the microelectrode points are made from a metal material, especially a metal material with good biocompatibility, such as Au and Pt, which can improve the signal collection efficiency. The wire on the microprobe may be prepared by a silicon-based process or other semiconductor processes, which facilitates integration and compatibility with the chip. Meanwhile, internal dimensions may be on the order of micron and sub-micron, and thus, compared with conventional millimeter-scale wires, an overall dimension may be reduced by 2~4 orders of magnitude, and accordingly, the size of product is reduced, and the manufactured microprobe is smaller and can be applied to implantation operation in a narrow space.

Considering that the number of microelectrode points on the microelectrode 1 is large, the plurality of microelectrode points are connected to wires and then connected to a back-end indium bump, and the wires need to be electrically isolated from each other, so as to prevent short circuit.

In some embodiments of the present application, the attachment unit is made of silica gel, preferably having a thickness of 0.1∼0.8 mm, but not limited to this thickness. The attachment unit made of silica gel, which is a flexible material, is attached to the posterior wall of the eyeball, which can achieve a better degree of attachment.

The above contents are further detailed description of the present application in conjunction with specific preferred embodiments, and cannot be considered that the specific implementation of the present application is only limited to the description. Those ordinarily skilled in the art to which the present application pertains, on the premise of not departing from the inventive concept of the present application, could further make a number of simple deductions or replacements, all of which should be considered within the scope of protection of the present application.

## Claims

1. A microneedle, comprising at least one microprobe, wherein the at least one microprobe each comprises a lining plate of an arc-shaped structure and at least one microelectrode.

2. The microneedle according to claim 1, wherein the microneedle comprises at least one integrated circuit chip, and the at least one microprobe and the at least one integrated circuit chip are in one-to-one correspondence.

3. The microneedle according to claim 2, wherein when the microprobe is in number of at least two, the at least one integrated circuit chip is located between two adjacent microprobes.

4. The microneedle according to claim 2, wherein the at least one integrated circuit chip is each provided thereon with at least one welding spot, the lining plate is provided thereon with at least one welding spot, and the at least one integrated circuit chip and the corresponding lining plate are bonded through the at least one welding spot.

5. The microneedle according to claim 1, wherein the microelectrode is a Utah electrode, a Michigan electrode or an optogenetic electrode.

6. The microneedle according to claim 1, wherein the microelectrode is located at an inner side of the lining plate.

7. The microneedle according to claim 1, wherein the microelectrode is provided thereon with at least one microelectrode point.

8. A neural interface system, comprising the microneedle according to any one of claims 1~7 and an attachment unit, wherein
the microneedle is configured for stimulating an optic nerve and/or collecting an optic nerve signal; and
the attachment unit is attached to a posterior wall of an eyeball.

9. The neural interface system according to claim 8, wherein the microneedle and the attachment unit are connected by a connecting line.

10. The neural interface system according to claim 8, wherein the attachment unit is made from a biocompatible material.
